# EUROPÄISCHE PATENTSCHRIFT

(11) **EP 0 328 968 B1**
(45) Veröffentlichungstag und Bekanntmachung des Hinweises auf die Patenterteilung: **12.05.1993**
(21) Anmeldenummer: 89101900.2
(22) Anmeldetag: 03.02.1989
(51) Int. Cl.: C07C 231/02, C07C 233/43, C07C 233/25

(54) **Verfahren zur Herstellung von 4-Acylamino-2-amino-alkoxy-benzolen**
Process for the preparation of 4-acylamino-2-amino alkoxy benzenes
Procédé pour la préparation de 4-acylamino-2-amino-alcoxy-benzènes

(30) Priorität: 13.02.1988 DE 3804621
(43) Veröffentlichungstag der Anmeldung: 23.08.1989
(73) Patentinhaber: HOECHST AKTIENGESELLSCHAFT, 65926 Frankfurt am Main (DE)
(72) Erfinder: Heise, Hartmut, Dr., D-6232 Bad Soden am Taunus (DE); Hintzmann, Manfred, Dr., D-6238 Hofheim am Taunus (DE)

(56) Entgegenhaltungen:
- EP-A- 0 011 048
- EP-A- 0 165 564
- FR-A- 2 359 815
- US-A- 3 928 451

## Beschreibung

Gegenstand der Erfindung ist ein gegenüber dem Stand der Technik verbessertes Verfahren zur Herstellung von 4-Acylamino-2-amino-alkoxybenzolen durch katalytische Hydrierung von 2,4-Dinitro-alkoxybenzolen in Butylacetat am Nickelkontakt zu 2,4-Diamino-alkoxybenzolen und deren anschließende selektive Acylierung.

Die Herstellung von 4-Acylamino-2-amino-alkoxybenzolen aus 2,4-Dinitro-alkoxybenzolen ist im Prinzip bekannt (EP 0011048). Die dort beschriebene Reduktion (Hydrierung) der Dinitroalkoxybenzole wird in alkoholischer Suspension in einer Wasserstoffatmosphäre in Gegenwart von Edelmetall-Katalysatoren durchgeführt. Die Beherrschung dieser stark exothermen Reaktion ist schwierig, zumal zur Vermeidung der Bildung teerartiger Nebenprodukte eine Temperatur von 60°C nicht überschritten werden sollte. Will man dieses Temperaturlimit einhalten, so darf die Reduktion nur langsam ablaufen, da es andernfalls nicht möglich ist, die freiwerdende Reduktionswärme von 2 Nitrogruppen abzuführen. Das bedeutet lange Reaktionszeiten und damit eine lange Lebensdauer von Reduktionszwischenstufen, die ihrerseits wegen der räumlichen Nähe der beiden reagierenden Substituenten in Reaktion treten können und zu einer vermehrten Bildung von Nebenprodukten führen.

Ebenfalls bekannt ist, daß die Lebensdauer von Reduktionszwischenstufen und damit die Möglichkeit zur Bildung unerwünschter Nebenprodukte vom verwendeten Lösungsmittel beeinflußt wird [JACS 74 (1952) 1297]. So hat sich gezeigt, daß Wasser als Lösungsmittel zur Reduktion (Hydrierung) von 2,4-Dinitrobenzolverbindungen sehr schlecht geeignet ist.

Bessere Ergebnisse werden mit Alkoholen, wie Methanol oder Ethanol, erzielt. Als Lösungsmittel besonders bewährt haben sich Essigsäurealkylester. Bei katalytischen Reduktionen (Hydrierungen) in ihnen werden die geringsten Anteile an Nebenprodukten beobachtet. So sind besonders Ethylacetat allein oder im Gemisch mit Alkoholen, wie Methanol oder Ethanol, als Lösungsmittel für katalytische Reduktionen von 2,4-Dinitrotoluol verwendet worden (DE-OS 24 28 141).

Ebenfalls bekannt ist die Verfahrensvariante, bei welcher man die Lebensdauer von Reduktionszwischenstufen dadurch verkürzt, daß man zu einer im Autoklav unter Wasserstoffatmosphäre vorgelegten und gerührten Katalysatorsuspension die Lösung bzw. Suspension der Dinitrobenzolverbindung pumpt (DE-OS 27 32 409; US-PS 3 154 584).

Als Katalysatoren zur Reduktion (Hydrierung) aromatischer 2,4-Dinitrobenzolverbindungen sind fast ausschließlich Edelmetall-Katalysatoren, wie Platin oder Palladium auf Kohle-Trägern oder Raney-Nickel verwendet worden, da nur derartige Katalysatoren Reduktionen (Hydrierungen) bei niedrigen Temperaturen erlauben (EP 0011048; JP-OS 81/45 445). Verwendet man dagegen Nickelkatalysatoren auf Kieselgurträgern, so sind erheblich höhere Hydriertemperaturen erforderlich, was speziell bei Dinitroverbindungen wegen der gegenseitigen Wechselwirkung der Reduktionszwischenstufen beim herkömmlichen Hydrierverfahren zu einem deutlich höheren Anteil an Nebenprodukten und damit zu einer Erniedrigung der Ausbeute führt.

Die sich an die katalytische Reduktion (Hydrierung) anschließende selektive Acylierung ist ebenfalls beschrieben worden (EP 0011048; GB-PA 1 324 303; JA-OS 78/15 328; JA-OS 80/167 264; JA-OS 81/45 445; JA-OS 84/65 054). Diamino-alkoxybenzol und Acylierungsmittel werden dabei stets in äquimolarem Verhältnis oder mit einem Überschuß an Acylierungsmittel eingesetzt. Als Zusätze, welche die Selektivität günstig beeinflussen sollen, werden u.a. Magnesiumoxid oder ein Gemisch aus Dialkylamin und Essigsäure verwendet. Die Acylierung wird dabei in dem Lösungsmittel, in welchem die katalytische Reduktion (Hydrierung) stattgefunden hat, durchgeführt.

Bei der Acylierung von 2,4-Diaminophenylethern werden nicht nur die gewünschten 4-Acylamino-2-aminophenylether gebildet, sondern das Reaktionsgemisch enthält daneben immer Anteile von nicht umgesetzter Ausgangsverbindung und von bis-acylierter Verbindung.

Es wurde nun überraschenderweise gefunden, daß man 4-Acylamino-2-amino-alkoxybenzole der allgemeinen Formel (1) 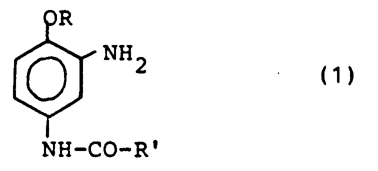
in welcher R eine Alkyl_{C₁-C₆}- oder Alkoxy_{C₁-C₄}-alkylen_{C₁-C₄}-Gruppe und R′ eine Methyl- odr Ethylgruppe bedeuten, in vorteilhafter Weise in höherer Ausbeute und besserer Qualität herstellen kann, indem man eine Lösung oder Suspension eines 2,4-Dinitro-alkoxybenzols der allgemeinen Formel (2) 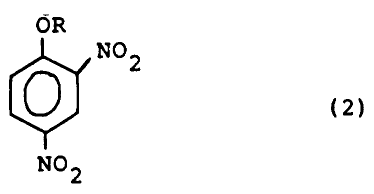
in welcher R die vorstehend genannte Bedeutung hat, in einem Butylacetat in eine in einem Autoklav vorgelegte und gerührte Suspension eines Nickelkatalysators auf einem Kieselgurträger in Butylacetat bei einem Wasserstoffdruck von 5 bis 50 bar, vorzugsweise 30 bis 40 bar, und einer Temperatur von 60 bis 120°C, vorzugsweise 80 bis 110°C, mit einer Geschwindigkeit pumpt, die der Geschwindigkeit der Hydrierung des 2,4-Dinitro-alkoxybenzols zum 2,4-Diamino-alkoxybenzol entspricht, nach erfolgter Hydrierung die Reduktionslösung azeotrop entwässert und das angefallene 2,4-Diaminoalkoxybenzol mit dem Anhydrid einer Alkylmonocarbonsäure von 2 bis 3 Kohlenstoffatomen bei einer Temperatur von -5 bis +15°C, vorzugsweise 5 bis 10°C, acyliert.

Was die beiden Reaktionsstufen hinsichtlich überraschendem Ergebnis anbelangt, so ist es als überraschend zu erachten, daß die Verwendung von Butylacetat als Lösungs- oder Verdünnungsmittel in Verbindung mit der Dosierung der Lösung oder Suspension des 2,4-Dinitro-alkoxybenzols zu der gerührten und unter Wasserstoffatmosphäre stehenden Katalysatorsuspension zu einer deutlich verbesserten Qualität und Ausbeute an 2,4-Diamino-alkoxybenzol bzw- 4-Acylamino-2-amino-alkoxybenzol führt, wobei die Dosiergeschwindigkeit der Hydriergeschwindigkeit entsprechen soll, d.h. die Reduktion (Hydrierung) zum Diamin soll spontan erfolgen und Reduktionszwischenstufen sollen so schnell durchlaufen werden, daß gegenseitige Wechselwirkungen ausgeschlossen sind.

Es ist ferner als überraschend anzusehen, daß diese Vorteile mit einem Nickelkatalysator auf einem Kieselgur-Träger zu erzielen sind, und zwar bei Temperaturen zwischen etwa 60 und etwa 120°C, was bei herkömmlichen Hydrierverfahren unter Verwendung von beispielsweise Alkohol oder Wasser als Lösungsmittel zu unbrauchbaren Produkten führt. Es hat sich nämlich gezeigt, daß das 2,4-Diamino-alkoxybenzol, welches bei der erfindungsgemäßen Ausführungsform spontan gebildet wird, im Hydriergemisch unter Wasserstoffatmosphäre selbst bei höheren Temperaturen sehr stabil ist.

Ferner haben Nickelkatalysatoren (auf einem Kieselgurträger) gegenüber Edelmetallkatalysatoren und Raney-Nickel den Vorteil, daß sie nicht selbstentzündlich und in der Praxis leicht und sicher zu handhaben sind. Darüber hinaus sind sie weniger empfindlich gegenüber Verunreinigungen und Katalysatorgiften und es kann bei ihnen aufgrund ihres vergleichsweise niedrigen Preises auf eine mehrmalige Verwendung verzichtet werden. Eine mehrmalige Verwendung, beispielsweise bei Edelmetallkatalysatoren, erfordert eine ständige Anpassung der Hydrierbedingungen an die von Einsatz zu Einsatz sinkenden Katalysatoraktivitäten. Dagegen ergibt sich bei einmaliger Verwendung des Katalysators der für die praktische Durchführung des Verfahrens große Vorteil der stets gleichbleibenden Reaktionsbedingungen.

Die sich an die katalytische Reduktion anschließende selektive Acylierung wird zweckmäßigerweise in dem gleichen Lösungsmittel vorgenommen, in welchem die katalytische Reduktion vorgenommen wurde (Butylacetat). Da jedoch bei der katalytischen Reduktion (Hydrierung) pro Mol 2,4-Dinitro-alkoxybenzol 4 Mol Wasser entstehen, und da Wasser die Acylierung hinsichtlich Ausbeute und Selektivität beeinträchtigt, ist es zweckmäßig, die angefallene Reduktionslösung vorher zu entwässern. Bei der Verwendung von Alkoholen als Lösungsmittel (EP 0011048; GB-PS 1 324 303) ist eine Entwässerung der Hydrierlösung praktisch nicht möglich. Setzt man jedoch Butylacetat als Lösungsmittel ein, so läßt sich das bei der Reduktion (Hydrierung) gebildete Wasser entweder als untere Phase aus dem Gemisch abtrennen oder durch azeotrope Destillation bequem entfernen, wobei der Entfernung durch azeotrope Destillation wegen der dadurch erzielbaren höheren Ausbeute der Vorzug zu geben ist. Auf die Verwendung von Magnesiumoxid wie bei dem in Alkohol durchgeführten Verfahren kann beim erfindungsgemäßen Verfahren verzichtet werden.

Überraschend bei der selektiven Acylierung ist ferner, daß ein bis zu 10 %iger Unterschuß an Acylierungsmittel, bezogen auf das 2,4-Diamino-alkoxybenzol, zu höheren Ausbeuten an 4-Acylamino-2-amino-alkoxybenzol führt. Eine darüber hinausgehende Zugabe an Acylierungsmittel bewirkt nicht nur keine Mehrbildung an Zielprodukt, sondern es erfolgt eine Abnahme der Ausbeute zugunsten der Bildung von 2,4-Bis-(acylamino)-alkoxybenzol.

Das erfindungsgemäße Verfahren wird im einzelnen wie folgt durchgeführt:
Zu einer Suspension des Nickel-Katalysators (auf einem Kieselgurträger) in Essigsäure-n-butyl- oder -iso-butylester oder in einer Mischung daraus, die in einem Autoklav unter einem Wasserstoffdruck von 5 bis 50 bar, vorzugsweise 30 bis 40 bar, vorgelegt und auf 80°C erwärmt wurde, pumpt man eine Lösung oder Suspension des 2,4-Dinitroalkoxybenzols in dem gleichen Lösungsmittel, welches zur Anschlämmung des Katalysators verwendet wurde, mit einer Geschwindigkeit, die der Hydriergeschwindigkeit der Dinitro- zur Diamino-Verbindung entspricht, was sehr leicht durch die Wasserstoffaufnahme kontrolliert werden kann. Im Verlauf der Reduktion steigt die Temperatur auf 110°C an. Der zur Verwendung gelangende Katalysator besteht aus einem Kieselgur-Träger mit bis zu 60 Gew.-% Nickel und wird vorzugsweise in einer Menge von 1 bis 3 Gew.-%, bezogen auf 2,4-Dinitro-alkoxybenzol eingesetzt.

Vor der in der zweiten Reaktionsstufe ohne Zwischenisolierung des erhaltenen 2,4-Diamino-alkoxybenzols erfolgenden selektiven Acylierung wird die vom Katalysator geklärte Reduktionslösung durch einfache azeotrope Destillation (Auskreisen) entwässert. Als Acylierungsmittel kommen Essigsäure- oder Propionsäureanhydrid in einer Menge von 0,90 bis 0,99 Mol, vorzugsweise 0,93 bis 0,96 Mol pro Mol 2,4-Diamino-alkoxybenzol zur Anwendung. Die Acylierung findet bie Temperaturen von -5 bis +15°C, vorzugsweise bei +5 bis +10°C, statt.

Die Verfahrensprodukte der genannten allgemeinen Formel (1) stellen wertvolle Kupplungskomponenten zur Herstellung von Dispersionsazofarbstoffen dar.

Durch die nachstehenden Beispiele soll das erfindungsgemäße Verfahren näher erläutert, nicht jedoch darauf beschränkt werden.

### Beispiel 1

In einen 2 l-Hydrierautoklav werden 3 g Nickel-Katalysator (mit ca. 55 Gew.-% Nickel auf einem Kieselgur-Träger) in 300 ml Essigsäure-n-butylester eingebracht. Nach Spülen mit Stickstoff werden 30 bar Wasserstoff aufgedrückt und die Katalysatorsuspension unter Rühren auf 80°C erwärmt. Danach beginnt man mit dem Hinzupumpen einer Lösung von 242,2 g 2,4-Dinitro-methoxyethoxybenzol in 500 ml Essigsäure-n-butylester, und zwar mit einer Geschwindigkeit, die der Reduktionsgeschwindigkeit bis zum Diamin entspricht, was man daran erkennt, daß unmittelbar nach Unterbrechung der Dosierung die Wasserstoffaufnahme endet.

Während der ca. 90-minütigen Dosierzeit steigt die Temperatur trotz Kühlung von 80°C auf ca. 110°C. Nach beendeter Reduktion wird der Autoklavinhalt auf 40°C gekühlt und ausgetragen. Die vom Katalysator geklärte Reduktionslösung wird anschließend azeotrop entwässert, wobei man das während der Reduktion gebildete Wasser über einen Wasserabscheider auskreist.

Die so erhaltene wasserfreie Lösung des Diamins in Essigsäure-n-butylester wird unter Rühren auf 5°C abgekühlt. Innerhalb von 6 Stunden pumpt man 123 g Propionsäureanhydrid gleichmäßig hinzu und läßt die Temperatur während dieser Zeit auf 10°C ansteigen. Nach Aufarbeitung und Trocknung werden 229,7 g eines 98,6 %igen Produkts erhalten, was einer Ausbeute an 4-Propionylamino-2-amino-methoxyethoxybenzol von 95,2 % der Theorie entspricht.

Nach HPLC-Analyse beträgt der Gehalt an nicht acyliertem 2,4-Diamino-methoxyethoxybenzol 0,3 %, der an 2,4-Bis-(propionylamino)-methoxyethoxybenzol 0,7 %.

### Beispiel 2

Man verfährt, wie in Beispiel 1 beschrieben, mit dem Unterschied, daß zur Acylierung 96,5 g Essigsäureanhydrid anstelle von 123 g Propionsäureanhydrid verwendet werden.

Nach Aufarbeitung und Trocknung werden 215,1 g eines 98,3 %igen Produkts erhalten, was einer Ausbeute an 4-Acetylamino-2-amino-methoxyethoxybenzol von 94,4 % der Theorie entspricht.

Der Gehalt an nicht acyliertem 2,4-Diamino-methoxyethoxybenzol beträgt 0,8 %, der an 2,4-Bis-(acetylamino)-methoxyethoxybenzol 0,5 % (HPLC).

### Beispiel 3

Man verfährt, wie in Beispiel 1 beschrieben, mit dem Unterschied, daß 198 g 2,4-Dinitro-methoxybenzol anstelle von 242,2 g 2,4-Dinitro-methoxyethoxybenzol, und Essigsäureanhydrid anstelle von Propionsäureanhydrid verwendet werden.

Nach Katalysatorklärung und azeotroper Entfernung des Hydrierwassers werden 972 g einer 13,9 gew.-%igen Lösung von 2,4-Diamino-methoxybenzol in Butylacetat erhalten, was einer Ausbeute von 97,9 % der Theorie entspricht.

Analog Beispiel 1 werden innerhalb von 6 Stunden 95,0 g Essigsäureanhydrid in die auf 5°C gekühlte und gerührte Diamin-Lösung gepumpt. Nach Aufarbeitung und Trocknung erhält man 165,8 g 4-Acetylamino-2-amino-methoxybenzol, was einer Ausbeute von 92 % der Theorie entspricht. Der Gehalt an nicht acyliertem 2,4-Diamino-methoxybenzol beträgt 0,8 %, der Gehalt an 2,4-Bis-(acetylamino)-methoxybenzol 1,2 % (HPLC).

### Beispiel 4

Man verfährt, wie in Beispiel 1 beschrieben, mit dem Unterschied, daß 226,2 g 2,4-Dinitrophenyl-isopropylether anstelle von 242,2 g 2,4-Dinitro-methoxyethoxybenzol verwendet werden.

Nach Aufarbeitung und Trocknung erhält man 216,4 g eines 98,4 %igen Produktes (Fp. 88 - 89°C), was einer Ausbeute an 4-Propionylamino-2-aminophenyl-isopropylether von 95,8 % der Theorie entspricht.

Der Gehalt an nicht acyliertem 2,4-Diaminophenyl-isopropylether beträgt 0,4 %, der an 2,4-Bis-(propionylamino)-phenyl-isopropylether 0,7 % (HPLC).

## Patentansprüche

1. Verfahren zur Herstellung von 4-Acylamino-2-amino-alkoxybenzolen der allgemeinen Formel (1) 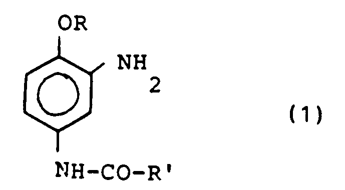 in welcher R eine Alkyl_{C₁-C₆}- oder Alkoxy_{C₁-C₄}-alkylen_{C₁-C₄}-Gruppe und R′ eine Methyl- oder Ethylgruppe bedeuten, dadurch gekennzeichnet, daß man eine Lösung oder Suspension eines 2,4-Dinitro-alkoxy-benzols der allgemeinen Formel (2) 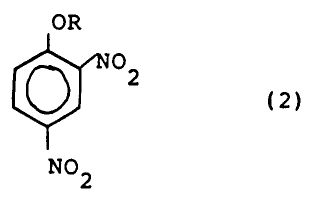 in welcher R die vorstehend genannte Bedeutung hat, in einem Butylacetat in eine in einem Autoklav vorgelegte und gerührte Suspension eines Nickelkatalysators auf einem Kieselgurträger in Butylacetat bei einem Wasserstoffdruck von 5 bis 50 bar und einer Temperatur von 60 bis 120°C mit einer Geschwindigkeit pumpt, die der Geschwindigkeit der Hydrierung des 2,4-Dinitro-alkoxybenzols zum 2,4-Diamino-alkoxybenzol entspricht, nach erfolgter Hydrierung die Reduktionslösung durch azeotrope Destillation entwässert und das erhaltene 2,4-Diamino-alkoxybenzol mit 0,90 bis 0,99 Mol des Anhydrids einer Alkylmonocarbonsäure von 2 bis 3 Kohlenstoffatomen, bezogen auf 1 Mol 2,4-Diamino-alkoxybenzol, bei Temperaturen von -5 bis +15°C acyliert.

2. Verfahren nach Anspruch 1, dadurch gekennzeichnet, daß man die Hydrierung und die Acylierung in Essigsäure-n-butylester oder Essigsäure-iso-butylester oder in einer Mischung daraus durchführt.

3. Verfahren nach mindestens einem der Ansprüche 1 und 2, dadurch gekennzeichnet, daß man die Hydrierung bei einem Wasserstoffdruck von 30 bis 40 bar durchführt.

4. Verfahren nach mindestens einem der Ansprüche 1 bis 3, dadurch gekennzeichnet, daß man die Hydrierung bei einer Temperatur von 80 bis 110°C durchführt.

5. Verfahren nach mindestens einem der Ansprüche 1 bis 4, dadurch gekennzeichnet, daß man die Acylierung bei Temperaturen von 5 bis 10°C vornimmt.

6. Verfahren nach mindestens einem der Ansprüche 1 bis 5, dadurch gekennzeichnet, daß man zur Acylierung das Alkylmonocarbonsäureanhydrid in einer Menge von 0,93 bis 0,96 Mol, bezogen auf 1 Mol 2,4-Diamino-alkoxybenzol, verwendet.

7. Verfahren nach mindestens einem der Ansprüche 1 bis 6, dadurch gekennzeichnet, daß man Essigsäureanhydrid als Acylierungsmittel verwendet.

8. Verfahren nach mindestens einem der Ansprüche 1 bis 6, dadurch gekennzeichnet, daß man Propionsäureanhydrid als Acylierungsmittel verwendet.

## Claims

1. A process for the preparation of 4-acylamino-2-amino-alkoxybenzenes of the formula (1) 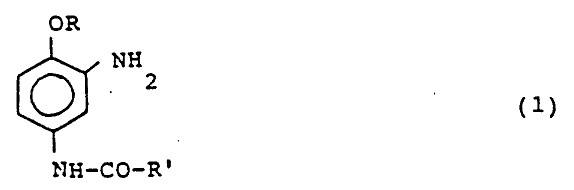 in which R denotes an alkyl-C₁-C₆- or alkoxy-C₁-C₄-alkylene-C₁-C₄ group and R' denotes a methyl or ethyl group, which comprises pumping a solution or suspension of a 2,4-dinitroalkoxybenzene of the formula (2) 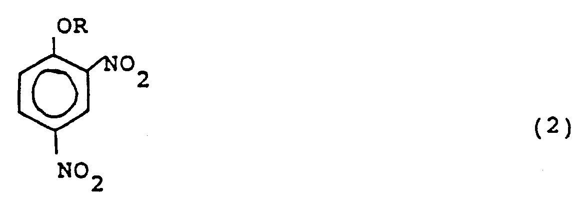 in which R has the abovementioned meaning, in a butyl acetate into a stirred suspension of a nickel catalyst on a kieselguhr carrier in butyl acetate, which has been initially introduced into an autoclave, at a hydrogen pressure of 5 to 50 bar, and a temperature of 60 to 120°C, at a rate which corresponds to the rate of the hydrogenation of 2,4-dinitroalkoxybenzene to 2,4-diaminoalkoxybenzene, dehydrating the reduction solution by azeotropic distillation, after the hydrogenation is completed, and acylating the resulting 2,4-diaminoalkoxybenzene with 0.90 to 0.99 mole of the anhydride of an alkylmonocarboxylic acid having 2 to 3 carbon atoms, relative to 1 mole of 2,4-diaminoalkoxybenzene, at temperatures of -5 to +15°C.

2. The process as claimed in claim 1, wherein the hydrogenation and the acylation is carried out in n-butyl acetate or iso-butyl acetate or in a mixture thereof.

3. The process as claimed in at least one of claims 1 and 2, wherein the hydrogenation is carried out at a hydrogen pressure of 30 to 40 bar.

4. The process as claimed in at least one of claims 1 to 3, wherein the hydrogenation is carried out at a temperature of 80 to 110°C.

5. The process as claimed in at least one of claims 1 to 4, wherein the acylation is carried out at temperatures from 5 to 10°C.

6. The process as claimed in at least one of claims 1 to 5, wherein the alkylmonocarboxylic anhydride for the acylation is used in an amount of 0.93 to 0.96 mole, relative to 1 mole of 2,4-diaminoalkoxybenzene.

7. The process as claimed in at least one of claims 1 to 6, wherein acetic anhydride is used as the acylating agent.

8. The process as claimed in at least one of claims 1 to 6, wherein propionic anhydride is used as the acylating agent.

## Revendications

1. Procédé de préparation de 4-acylamino-2-aminoalcoxybenzènes de formule générale 1 ci-dessous : 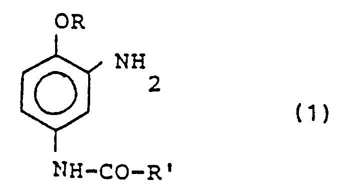 dans laquelle R représente un alkylC₁-C₆- ou alcoxy-C₁-C₄-alkylene-C₁-C₄ et R' le groupe méthyle ou éthyle, procédé caractérisé en ce que l'on pompe une solution ou une suspension d'un 2,4-dinitroalcoxybenzène de formule générale 2 : 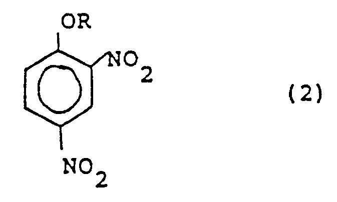 dans un acétate de butyle, dans une suspension, placée dans un autoclave et agitée, d'un catalyseur de nickel sur Kieselgur dans l'acétate de butyle, sous une pression d'hydrogène de 5 à 50 bar, et à une température de 60 à 120°C, à un débit correspondant à la vitesse d'hydrogénation du 2,4-dinitroalcoxybenzène en 2,4-diaminoalcoxybenzène, hydrogénation après laquelle on déshydrate par distillation azéotropique la solution de réduction puis on acyle le 2,4-diaminoalcoxybenzène formé avec de 0,90 à 0,99 mol de l'anhydride d'un acide alkylmonocarboxylique à deux ou trois atomes de carbone par mole du 2,4-diamino-alcoxybenzène, à des températures de -5 à +15°C.

2. Procédé selon la revendication 1, caractérisé en ce que l'on effectue l'hydrogénation et l'acylation dans de l'acétate de n-butyle ou d'isobutyle ou dans un mélange des deux.

3. Procédé selon l'une au moins des revendications 1 et 2, caractérisé en ce que l'on effectue l'hydrogénation sous une pression d'hydrogène de 30 à 40 bar.

4. Procédé selon l'une au moins des revendications 1 à 3, caractérisé en ce que l'on effectue l'hydrogénation à une température de 80 à 110°C.

5. Procédé selon l'une au moins des revendications 1 à 4, caractérisé en ce que l'on effectue l'acylation à des températures de 5 à 10°C.

6. Procédé selon l'une au moins des revendications 1 et 5, caractérisé en ce que l'on effectue l'acylation avec de 0,93 à 0,96 mol de l'anydride de l'acide alkylmonocarboxylique par mole du 2,4-diaminoalcoxybenzène.

7. Procédé selon l'une au moins des revendications 1 et 6, caractérisé en ce que l'on effectue l'acylation avec de l'anhydride acétique.

8. Procédé selon l'une au moins des revendications 1 et 6, caractérisé en ce que l'on effectue l'acylation avec de l'anhydride propionique.
